Europäisches Patentamt

European Patent Office

Office européen des brevets

(11) Publication number: **0 123 467**
**B1**

(12) **EUROPEAN PATENT SPECIFICATION**

(45) Date of publication of patent specification: **22.11.90**

(21) Application number: **84302348.2**

(22) Date of filing: **05.04.84**

(51) Int. Cl.[5]: **B 01 J 27/28,** C 07 C 51/215, C 07 C 57/145

(54) Reactivation of phosphorus vanadium catalysts and process for the manufacture of maleic anhydride catalysts treated with alkyl esters of orthophosphoric acids in the presence of water.

(30) Priority: **14.04.83 US 484949**

(43) Date of publication of application:
**31.10.84 Bulletin 84/44**

(45) Publication of the grant of the patent:
**22.11.90 Bulletin 90/47**

(84) Designated Contracting States:
**AT BE CH DE FR GB IT LI LU NL SE**

(56) References cited:
**GB-A-1 464 198**
**US-A-3 474 041**
**US-A-4 418 003**

(73) Proprietor: **AMOCO CORPORATION**
**200 East Randolph Drive**
**Chicago Illinois 60601 (US)**

(72) Inventor: **Edwards, Robert Charles**
**1418 Lombardy Lane**
**Naperville Illinois 60540 (US)**
Inventor: **Kilner, Peter Hampton**
**5139 N.E. River Road Apt. 371**
**Chicago Illinois 60656 (US)**
Inventor: **Udovich, Carl Anthony**
**3526 Bakview Lane**
**Joliet Illinois 60435 (US)**
Inventor: **Stauffenberg, Dennis Lee**
**140 Jan Street, Box 587**
**Manhattan Illinois 60442 (US)**

(74) Representative: **Ritter, Stephen David et al**
**Mathys & Squire 10 Fleet Street**
**London EC4Y 1AY (GB)**

The file contains technical information
submitted after the application was filed and
not included in this specification

Courier Press, Leamington Spa, England.

**Description**

The field of the invention relates to reactivation of phosphorus-vanadium catalysts, with a hydrocarbon ester of phosphoric acid, in the presence of water and to the color stabilization of the maleic anhydride produced by the vapor phase oxidation of butane in the presence of the catalyst.

Maleic anhydride is of significant commercial interest throughout the world and is extensively used in the production of alkyl resins. It is also a versatile intermediate for chemical synthesis. Consequently, large quantities of maleic anhydride are produced each year to satisfy these needs. The production of maleic anhydride by the catalytic oxidation of benzene has, until recently, been the principal method for the manufacture of maleic anhydride. However, because of the inherent toxicity of benzene the trade has commenced to eliminate the utilization of benzene as a feedstock and newer facilities tend to utilize butane oxidation processes for the production of maleic anhydride.

In general, catalysts utilized for the oxidation of butane to maleic anhydride are based upon vanadium and phosphorus. Various metal activators have been used to enhance the phosphorus-vanadium catalyst. The difficulty with the phosphorus-vanadium metal promoted catalyts is that they tend to deactivate quite quickly. In this connection, U.S. Patents 4,002,174; 4,094,816 and 4,089,807 teach that carbon tetrachloride can be used to improve the vanadium-phosphorus metal promoted catalyst reactivation. In U.S. Patent 3,296,282 and U.S. Patent 3,474,041 there is desribed a method for the regeneration of vanadium-phosphorus oxidation catalysts used in the oxidation of olefins to make maleic anhydride. These references disclose the processs of treating the catalyst with a phosphine, phosphite or phosphonate by periodically or continuously passing the phosphorus compound to the reactor, with or without interrupting the olefin feed flow.

US—A—4418003 describes a procedure for manufacturing phosphorus-vanadium catalysts suitable for the manufacture of maleic anhydride by reaction $P_2O_5$ and a vanadium compound in an organic medium, e.g. an aliphatic alcohol. The use of $P_2O_5$ enables mixed phosphate esters to be formed which are advantageously incorporated within the catalyst precursors. Catalyst regeneration is not specifically discussed.

United Kingdom Patent Specifiocation GB—A—1,464,198 teaches regeneration of phosphorus complexes with certain phosphates. This reference refers to the use of steam as a carrier for adding the phosphate ester but does not disclose the continuous regeneration of vanadium-phosphorus, or vanadium-phosphorus-co-metal catalyst by organic phosphate in the presence of from 1 to 50 weight percent water in the feedstream. Further, this reference does not suggest that the phosphates used in regeneration improved the color stability of the resulting maleic anhydride.

We have now discovered a method for regenerating *in situ* vanadium-phosphorus and vanadium-phosphorus-co-metal catalyst complexes used in the vapor phase oxidation of butane to maleic anhydride.

In accordance with the invention there is provided a continuous process for the vapor phase oxidation of butane feedstock to form maleic anhydride in which butane is contacted in the presence of molecular oxygen or air with a vanadium-phosphorus-oxygen-water catalyst wherein the catalyst is regenerated by contacting it with an alkyl ester of orthophosphoric acid having the formula $(RO)_3P=O$ where R is hydrogen or a $C_1$ to $C_4$ alkyl, at least one R being a $C_1$ to $C_4$ alkyl, characterised in that the catalyst is regenerated continously by contacting it during the vapor phase oxidation with said alkyl ester of orthophosphoric acid and added water wherein the amount of water added is from 1 to 50 weight percent of the catalyst feedstream.

By regenerating the catalyst continuously, significantly better maleic anhydride yields are obtained. Also improved color and color stability of maleic anhydride produced can be achieved.

According to our process, the vapor phase oxidation of butane to maleic anhydride may be conducted by contacting the butane in the presence of a vanadium-phosphorus-oxygen catalyst or a catalyst promoted with metals. Suitable co-metals are zinc, molybdenum, zirconium, niobium, cerium, chromium, manganese, nickel and uranium. The preferred co-metals are molybdenum and zinc.

The catalyst to be reactivated can be prepared in various ways including the one disclosed in U.S. Patent 3,862,146, issued January 21, 1975, having Edward M. Boghosian as its inventor. The catalyst prepared according to the process disclosed in U.S. Patents 4,418,003; 4,416,802 and 4,416,803. Alternatively, the catalyst to be reactivated can suitably be prepared from an alcohol solution which has been reacted with phosphoric pentoxide and has been saturated with an inorganic acid, such as hydrogen chloride. Other ways to prepare the catalyst are disclosed in U.S. Patent 4,328,126 wherein the catalyst is made from an organic solvent system. Precipitation of the phosphorus-vanadium-zinc mixed oxide can suitably be effected by azeotropic distillation of the organic solvent and the water of reaction and the subsequent evaporation of the organic solvent. The atomic ratio of vanadium to phosphorus can suitably be in the range of about 0.5:1 to about 1.25:1, preferably, in the range of about 0.6:1 to about 1:1. The total atomic ratio of zinc or molybdenum to vanadium is advantageously in the range of about 0.005:1 to about 0.25:1. The atomic ratio of phosphorus to vanadium is suitably in the range of about 2:1 to about 0.8:1, preferably about 1:1 to about 1.7:1. The reactivation of the catalyst can also be suitably conducted by dissolving the alkyl ester of phosphoric acid in water and applying this solution in a uniform manner to the catalyst to be regenerated. This method is particularly suitable in continuous processes which utilize multi-tubular upflow reactors. In this process, the alkyl ester, in an aqueous medium comprising about 0.01 to

2

about 100 weight percent, more preferably about 25 to 75 weight percent of the alkyl ester, is sprayed as a liquid into the feed gas stream flowing to the reactor. This process has great advantages over conventional additions of regenerating agents, which entail plant shutdowns, since, in our novel process, the reactivation is conducted *in situ* without interrupting production or utilizing a hot oil vaporizer which tends to decompose alkyl phosphates.

The continuous reactivation is applicable to phosporus-vanadium catalysts and to phosphorus-vanadium catalysts promoted by metals, which are disclosed hereinabove. Suitable metals include molybdenum, zinc, tungsten, tin, and bismuth, etc.

The reaction may be conducted at a temperature of about 650° to about 900°F (343—482°C). The alkyl phosphate in a water medium comprising about 0.001 to about 100 weight percent, more preferably about 0.001 to about 50 weight percent of the solution, is contacted with the feed gas stream flowing to the reactor. Alternatively, the alkyl phosphate and water may be added directly to the butane feed prior to the mixing of the butane and air reactants. The oxidation of butane to maleic anhydride may be accomplished by contacting n-butane in low concentration in oxygen with the described catalyst. Air is entirely satisfactory as a source of oxygen, but synthetic mixtures of oxygen and diluent gases such as nitrogen may also be employed. Air enriched with oxygen may be used.

The gaseous feedstream to the oxidation reactors will normally contain air and about 0.2 to about 1.7 mole percent of n-butane. About 0.8 to about 1.5 mole percent of n-butane is satisfactory for optimum yield of maleic anhydride or the process of this invention. Although higher concentrations may be employed, explosive hazards may be encountered. Lower concentrations of butane, less than about one percent, of course, will reduce the total yield obtained at equivalent flow rates and, thus, are not normally employed for economic reasons. The flow rate of the gaseous stream through the reactor may be varied within rather wide limits, but preferred range of operations is at the rate of about 100 to about 4000 cc of feed per cc of catalysts per hour and more preferably about 1000 to about 2400 cc of feed per cc of catalyst per hour. Residence times of the gas stream will normally be less than about four seconds, more preferably less than about one second, and down to a rate where more efficient operations are obtained. The flow rates and residence times are calculated at standard conditions of about 760 mm of mercury and at about 25°C. A variety of reactors will be found to be useful and multiple tube heat exchanger type reactors are quite satisfactory. The tops of such reactors may vary in diameter from about one-quarter inch to about three inches, and the length may be varied from about three to about ten or more feet. The oxidation reaction is an exothermic reaction and, therefore, relatively close control of the reaction temperatures should be maintained. It is desirable to have the surface of the reactors at a relatively constant temperature and some medium is needed to conduct heat from the reactors, such as lead and the like, but it has been found that eutectic salt baths are completely satisfactory. One such salt bath is a sodium nitrate, sodium nitrite-potassium nitrate eutectic constant temperature mixture. An additional method of temperature control is to use a metal block reactor whereby the metals surrounding the tube act as a temperature regulating body. As will be recognized by one skilled in the art, the heat exchanger medium may be kept at the proper temperature by heat exchangers and the like. The reactor or reaction tubes may be iron, stainless steel, carbon steel, nickel, glass tubes such as vycor and the like. Both carbon steel and nickel tubes have excellent long life under the conditions of the reaction described herein. Normally, the reactors contain a preheat zone under an inert material such as one-quarter inch Alundum pellets, inert ceramic balls, nickel balls, or chips and the like present at about one-half to one-tenth the volume of the active catalyst present.

The temperature of reaction may be varied within some limits, but normally the reaction should be conducted at a temperature within a rather critical range. The oxidation reaction is exothermic and once reaction is underway, the main purpose of the salt bath or other media is to conduct heat away from the walls of the reactor and control the reaction. Better operations are normally obtained when the reaction temperature employed is no greater than about 20° to about 50°F (11—28°C) above the salt bath temperature. The temperature of the reactor, of course, will also depend to some extent upon the size of the reactor and the butane concentration.

The reaction may be conducted at atmospheric, super-atmospheric or below atmospheric pressure. The exit pressure will be at least slightly higher than the ambient pressure to ensure a positive flow from the reaction. The pressure of the inert gases must be sufficiently higher to overcome the pressure drop through the reactor.

Maleic anhydride may be recovered by a number of ways well known to those skilled in the art. For example, the recovery may be direct condensation or by absorption in suitable media, with specific operation and purification of the maleic anhydride. The following examples will serve to provide a fuller understanding of the invention, but it is to be understood that these examples are given for illustrative purposes only and should not be interpreted as limiting the invention in any way. In the examples, the term "conversion", "selectivity" and "yield" are defined as follows:

$$\text{Conversion \%} = \frac{\text{Mole n-butane reacted}}{\text{Moles n-butane in feed}} \times 100$$

$$\text{Selectivity \%} = \frac{\text{Moles maleic produced}}{\text{Moles n-butane feed consumed}} \times 100$$

$$\text{Yield Wt.\%} = \text{(Conversion)} \times \text{(Selectivity)} \times 169$$

## Example 1

A vanadium-oxygen-phosphorus catalyst promoted with Zn gave a yield of 87 wt.% at 792°F (422°C), 2000 volume hourly space velocity (VHSV), and 1.5% n-butane. The feed contained 10,000 ppm water at this time. After 524 additional hours on stream, at the above conditions, and at 780°F, the catalyst gave an 80 wt.% yield of maleic anhydride. Triethylphosphate, 1.6 ppm, was added to the feed stream 48 hours later. After another 192 hours, the yield has improved to 85 wt.% at 799°F (426°C) and the same conditions. The water was removed from the feed stream 521 hours later while continuing with 1.4 ppm of triethylphosphate addition. After another 160 hours the yield declined to 73 wt.% at 779°F (415°C) and the same flow conditons. Upon increasing the triethylphosphate addition to 3.2 ppm 194 hours later, the yield declined further. 336 hours after this, the yield was only 55 wt.% at 780°F (416°C) and the same flow conditions. Water was reintroduced after 24 hours at 10,000 ppm and the alkylphosphate addition was dicontinued. After 120 hours the yield had improved to 77 wt.%. This example illustrates that a combination of water and alkylphosphate ester is necessary for the optimum regeneration of catalysts. Note that without water addition the alkylphosphate addition actually resulted in a yield decline with this particular catalyst.

## Example 2

A used phosphorus-oxygen-vanadium catalyst gave a yield of 55 wt.% at 782°F (417°C), 2000 VHSV, and 1.5% n-butane at 115 hours on stream in a pilot plant. Addition of 1.6—5.0 ppm of triethylphosphate to the feed stream produced a maximum yield of 71 wt.% at 813°F (434°C) and the same flow conditions at 1090 hours on stream. The yield then declined to 61 wt.% at 2496 hours on stream at 818°F (437°C) with 7.4 ppm of triethylphosphate addition. The phosphate addition was then discontinued after 2497 hours. After 3048 hours the yield was 58 wt.% at 797°F (425°C), 2000 VHSV, and 1.5% n-butane. Water was then added to the feed at a concentration of 10,000 ppm along with 1.5—13 ppm of triethylphosphate. After 4756 hours on stream this catalyst gave a maximum yield of 73.6 wt.% at 845°F (452°C), 2000 VHSV, 1.5% n-butane, 10,000 ppm of water, and 13 ppm of triethylphosphate. In this example, the alkylphosphate ester produced a yield increase without water present. Then the treatment resulted in a yield decline. However, adding the alkylphosphate with water produced a higher maximum yield and continued treatment gave no yield decline.

Prolonged addition of phosphate without the co-addition of water results in a yield decline. This decline is dependent on the catalyst used. Again, the use of water and alkylphosphate esters gives better results when compared to the use of alkylphosphates without water.

## Example 3

A phosphorus-vanadium-oxygen catalyst containing no co-metal or other promoter was prepared according to the method reported in U.S. 4,418,003. A 6 cc sample, 5.60 g, of this catalyst in the form of 3/16 inch by 3/16 inch (5 mm × 5 mm) cylindrical pellets was loaded into a 0.62 inch diameter minireactor. A 1.08 mole % n-butane in air mixture was passed through a saturator containing water and then over the catalyst at 1200 VHSV and 730—750°F (388—399°C). In this manner, about 10,000 ppm of water are continuously added to the reactor feedstream.

This catalyst gave a maximum yield of 96 wt.% at 1200 VHSV and 731°F after three days on stream. The yield declined to 85 wt.% at 1200 VHSV and 738°F (392°C) after 21 days on stream. At this time the feedstream was passed through a saturator containing a 20 wt.% aqueous solution of triethylphosphate. After 28 days on stream the yield improved to 88 wt.% at 1200 VHSV and 739°F (393°C). This example illustrates that triethylphosphate and water can regenerate a phosphorus-vanadium-oxygen catalyst.

## Claims

1. A continuous process for the vapor phase oxidation of butane feedstock to form maleic anhydride in which butane is contacted in the presence of molecular oxygen or air with a vanadium-phosphorus-oxygen-water catalyst wherein the catalyst is regenerated by contacting it with an alkyl ester of orthophosphoric acid having the formula $(RO)_3P{=}O$ where R is hydrogen or a $C_1$ to $C_4$ alkyl, at least one R being a $C_1$ to $C_4$ alkyl, characterised in that the catalyst is regenerated continously by contacting it during the vapor phase oxidation with said alkyl ester of orthophosphoric acid and added water wherein the amount of water added is from 1 to 50 weight percent of the catalyst feedstream.

EP 0 123 467 B1

2. A process according to Claim 1 wherein the alkyl ester is mixed with the butane feedstock prior to oxidation.

3. A process according to Claim 1 or Claim 2 wherein the alkyl ester is triethylphosphate.

4. A process according to Claim 1 or Claim 2 wherein the alkyl ester is trimethylphosphate.

5. A process according to any preceding claim wherein the reaction temperature is about 650°F to about 900°F (343—482°C).

6. A process according to any preceding claim wherein a fixed bed catalyst is used and the feedstock contains about 0.2 to about 1.7 mole per cent butane.

7. A process according to any preceding claim wherein a vanadium phosphorus oxide promoter catalyst is reactivated.

8. A process according to any preceding claim wherein the catalyst comprises a vanadium-phosphorus-oxygen-water catalyst promoted by a metal.

9. A process according to any preceding claim wherein the catalyst comprises a vanadium-phosphorus-oxygen-water catalyst promoted by zinc or molybdenum.

10. A process according to any preceding claim which comprises applying continuously about $4 \times 10^{-4}$ to about 10 grams of an alkyl ester of orthophosphoric acid having the formula $(RO)_3P=O$ where R is hydrogen or a $C_1$ to $C_4$ alkyl, at least one R being a $C_1$ to $C_4$ alkyl per kilogram of the catalyst per hour and water and wherein the amount of water added is about $1 \times 10^{-5}$ to about 0.1 kilograms per hour per kilogram of catalyst.

**Patentansprüche**

1. Kontinuierliches Verfahren zur Dampfphasenoxidation einer Butanbeschickung unter Bildung von Maleinsäureanhydrid, durch Behandlung von Butan in Anwesenheit von molekularem Sauerstoff oder Luft mit einem Vanadium-Phorphor-Sauerstoff-Wasser-Katalysator, wobei dieser Katalysator durch Behandlung mit einem Orthophosphorsäurealkylester der Formel $(RO)_3P=O$ worin R Wasserstoff oder $C_1$—$C_4$-Alkyl ist, wobei wenigstens einer der Substituenten R für $C_1$—$C_4$-Alkyl steht, regeneriert wird, dadurch gekennzeichnet, daß der Katalysator durch kontinuierliche Behandlung während der Dampfphasenoxidation mit dem Orthophosphorsäurealkylester und unter Zusatz von Wasser regeneriert wird, wobei die zugesetzte Menge an Wasser 1 bis 5 Gewichtsprozent des Katalysatorbeschickungsstroms beträgt.

2. Verfahren nach Anspruch 1, worin der Alkylester vor der Oxidation mit der Butanbeschickung vermischt wird.

3. Verfahren nach Anspruch 1 oder 2, worin der Alkylester Triethylphosphat ist.

4. Verfahren nach Anspruch 1 oder 2, worin der Alkylester Trimethylphosphat ist.

5. Verfahren nach einem der vorhergehenden Ansprüche, worin die Reaktionstemperatur etwa 650°F bis etwa 900°F (343 bis 482°C) beträgt.

6. Verfahren nach einem der vorhergehenden Ansprüche, worin ein Festbettkatalysator verwendet wird und die Beschickung etwa 0,2 bis etwa 1,7 Molprozent Butan enthält.

7. Verfahren nach einem der vorhergehenden Ansprüche, worin ein Vanadium-Phosphoroxid-Promotorkatalysator reaktiviert wird.

8. Verfahren nach einem der vorhergehenden Ansprüche, worin der Katalysator ein durch ein Metall promovierter Vanadium-Phosphor-Sauerstoff-Wasser-Katalysator ist.

9. Verfahren nach einem der vorhergehenden Ansprüche, worin der Katalysator ein durch Zink oder Molybdän promovierter Vanadium-Phosphor-Sauerstoff-Wasser-Katalysator ist.

10. Verfahren nach einem der vorhergehenden Ansprüche, dadurch gekennzeichnet, daß etwa $4 \times 10^{-4}$ bis etwa 10 g eines Orthophosphorsäurealkylesters der Formel $(RO)_3P=O$, worin R Wasserstoff oder $C_1$—$C_4$-Alkyl ist, wobei wenigstens einer der Substituenten R für $C_1$—$C_4$-Alkyl steht, pro kg des Katalystors und pro Stunde sowie Wasser kontinierlich zugeführt werden, wobei die Menge an zugesetztem Wasser etwa $1 \times 10^{-5}$ bis etwa 0,1 kg pro Stunde und pro kg Katalysator beträgt.

**Revendications**

1. Procédé en continu pour l'oxydation en phase vapeur d'un substrat de butane pour former de l'anhydride maléique dans lequel le butane est mis en contact en présence d'oxygène moléculaire ou d'air avec un catalyseur vanadium-phosphore-oxygène-eau, le catalyseur étant régénéré pr sa mise en conctact avec un ester alcoylique de l'acide orthophosporique présentant la formule $(RO)_3P=O$ où R est hydrogène ou un alcoyle en $C_1$ à $C_4$, au moins un R étant un alcoyle en $C_1$ à $C_4$, caractérisé en ce que le catalyseur est régénéré en continu en le mettant en contact pendant l'oxydation en phase vapeur avec ledit ester alcoylique de l'acide orthophosphorique et de l'eau ajoutée, la quantité d'eau ajoutée étant de 1 à 50% en poids du courant d'alimentation du catalyseur.

2. Procédé selon la revendication 1, dans lequel l'ester alcoylique est mélangé avec le substrat de butane avant l'oxydation.

3. Procédé selon la revendication 1 ou la revendication 2, dans lequel l'ester alcoylique est le triéthylphospate.

5

4. Procédé selon la revendication 1 ou la revendication 2, dans lequel l'ester alcoylique est le triméthylphospate.

5. Procédé selon l'une quelconque des revendications précédentes, dans lequel la température de réaction est d'environ 650°F à environ 900°F (343—482°C).

6. Procédé selon l'une quelconque des revendications précédentes, dans lequel un catalyseur en lit fixe en utilisé et le substrat contient environ 0,2 à environ 1,7 mole pour cent de butane.

7. Procédé selon l'une quelconque des revendications précédentes, dans lequel un catalyseur promoteur oxyde de vanadium phosphore est réactivé.

8. Procédé selon l'une quelconque des revendications précédentes, dans lequel catalyseur comprend un catalyseur vanadium-phosphore-oxygène-eau activé par un métal.

9. Procédé selon l'une quelconque des revendications précédentes, dans lequel le catalyseur comprend un catalyseur vanadium-phosphore-oxygène-eau activé par le zinc ou le molybdène.

10. Procédé selon l'une quelconque des revendications précédentes, qui comprend l'application en continu d'environ $4 \times 10^{-4}$ à environ 10 grammes d'un ester alcoylique de l'acide orthophosphorique présentant la formule $(RO)_3P=O$ où R est hydrogène ou un alcoyle en $C_1$ à $C_4$, au moins un R étant un alcoyle en $C_1$ à $C_4$, par kilogramme du catalyseur par heure et de l'eau et dans lequel la quantité d'eau ajoutée est d'environ $1 \times 10^{-5}$ à environ 0,1 kilogramme par heure par kilogramme de catalyseur.

6